# EUROPEAN PATENT APPLICATION

(11) **EP 4 361 255 A1**
(43) Date of publication of application: **01.05.2024**
(21) Application number: 22828012.9
(22) Date of filing: 26.03.2022
(51) Int. Cl.: C12N 5/071, C12M 3/00, C12N 5/07, C12N 5/10

(54) **CELL CULTURE METHOD, CELL CULTURE KIT, AND CELL CULTURE SYSTEM**

(30) Priority: 25.06.2021 JP 2021105362
(71) Applicant: Toyo Seikan Group Holdings, Ltd., Shinagawa-ku Tokyo 141-8627 (JP)
(72) Inventor: NISHIYAMA, Takaharu, Yokohama-shi, Kanagawa 240-0062 (JP); TANAKA, Satoshi, Yokohama-shi, Kanagawa 240-0062 (JP); KOSEKI, Osamu, Yokohama-shi, Kanagawa 240-0062 (JP); TOTANI, Takahiko, Yokohama-shi, Kanagawa 240-0062 (JP)
(74) Representative: Hasegawa, Kan
(86) International application number: PCT/JP2022/014747
(87) International publication number: WO 2022/270093

(57) **Abstract**

Provided is a cell culture method that can suitably control the exchange rate of a medium inside a vessel in the cell culture using a culture vessel. The cell culture method uses a culture vessel and a medium supplying vessel. Cells and a medium are filled and cell culture is performed in the culture vessel, a new medium is filled in the medium supplying vessel, and the medium supplying vessel supplies the medium to the culture vessel. The culture vessel includes at least a first port used for medium supply and a second port used for medium discharge, and the medium supplying vessel serves as a mixing vessel for adjusting a concentration of the new medium to be supplied to the culture vessel. The cell culture method includes: transferring a portion of a used medium in the culture vessel from the culture vessel to the mixing vessel; creating a concentration-adjusted medium by mixing the new medium and the portion of the used medium in the mixing vessel; and filling the culture vessel with the concentration-adjusted medium by discharging a remaining used medium from the culture vessel while the concentration-adjusted medium is transferred from the mixing vessel to the culture vessel.

## Description

### TECHNICAL FIELD

The present invention relates to a cell culture technique, in particular relates to a medium exchange in cell culture using a culture vessel.

### BACKGROUND ART

In recent years, it has been required that cells, tissues, or the like are efficiently cultured in large amounts under artificial environment in fields of production of pharmaceutical medicines, gene therapy, regeneration therapy, immunotherapy, and the like.

In such a situation, in order to culture cells in large amounts, various methods for automatically culturing cells using a culture vessel have been developed.

When cell culture using a culture vessel is performed, a medium that has been used inside the vessel for a certain period of time needs to be exchanged for a new medium at a predetermined timing.

Examples of methods for exchanging a medium inside a vessel mainly include a circulation method in which the medium inside the vessel is exchanged by circulating it and a non-circulation method in which the medium inside the vessel is exchanged by transferring it in one direction without circulating it.

The circulation method here means a method in which, after discharging a certain ratio of a used medium from the culture vessel and supplying a new medium, the medium is circulated using a circulation passage in order to uniformly mix these mediums inside the culture vessel.

Patent Document 1: JP-A-2020-188691

### DISCLOSURE OF THE INVENTION

### PROBLEMS TO BE SOLVED BY THE INVENTION

However, in a circulation method, there is a problem in that increasing an exchange rate of a medium to nearly 100% requires very complicated work. Namely, the actual situation is that this circulation method is performed as follows.

First, when a used medium is discharged from a culture vessel, there is a problem that when the medium is completely discharged, cells will be discharged together with the medium. In view of this, it is necessary to cause a portion of the used medium to remain inside the vessel by discharging the medium in a state where a liquid thickness of the medium inside the vessel is maintained at a certain level or higher.

In particular, when spheres (cell aggregations) made of iPS cells or the like, organoid generated by aggregating several types of cells, or floating cells are cultured, since these are easily discharged when discharging the medium, and it is necessary to keep the liquid thickness thicker than when adherent cells are cultured by adhering them to an inner surface of the vessel, there is a problem that a medium exchange rate becomes low.

When a new medium is supplied into the vessel after discharging the used medium from the culture vessel, there is a problem in that the medium inside the vessel becomes non-uniform and culture efficiency decreases.

In view of this, it is necessary to perform the circulation method in which two ports are equipped in a culture vessel, and these ports are coupled with a tube to form a circular flow passage, and the medium inside the vessel is circulated to homogenize the medium, or to perform stirring and homogenizing the medium by applying vibration to the culture vessel, or the like.

Thus, when the circulation method is performed, in order to increase the exchange rate of the medium to nearly 100%, it is necessary to repeatedly discharge the used medium from the culture vessel, supply a new medium into the culture vessel, and perform the circulation method by forming a circular flow passage, which requires complicated work. Even in a case of applying vibration to the culture vessel to homogenize the medium, manual stirring, or use of stirring means is required, which is complicated.

On the other hand, in the non-circulation method, while it is possible to perform the medium exchange with an exchange rate of the medium of 100%, there is a problem in that it is difficult to control the exchange rate of the medium.

Namely, according to the non-circulation method, it is possible to exchange 100% of the medium by including two ports in the culture vessel, coupling a medium supply tube and a medium discharge tube to each port, and supplying a new medium from the medium supply tube while discharging the used medium from the medium discharge tube.

However, for example, when spheres are differentiated and induced into mesoderm, or the like, it has been experimentally found that when the medium is exchanged close to 100%, the culture efficiency of the cells is rather inhibited (see Reference Example 1). Accordingly, while it is desirable to be able to control the exchange rate of the medium to a desired level, rather than 100% only, this has been difficult to achieve with the non-circulation method. In the non-circulation method, there is also a problem that an amount of mediums required for the medium exchange has increased.

Therefore, the present inventors have conducted extensive research, have succeeded in developing a method that does not require formation of a circular flow passage or stirring of the culture vessel for homogenizing the medium inside the culture vessel and is capable of suitably controlling the exchange rate of the medium inside the vessel, and have completed the present invention.

Here, patent document 1 discloses a cell culture apparatus for continuously culturing cells and describes a configuration for controlling supply of a medium. However, the cell culture apparatus has not allowed suitable control of the exchange rate of the medium.

The present invention has been made in view of the above circumstances, it is an object of the present invention to provide a cell culture method, a cell culture kit, and a cell culture system that can suitably control the exchange rate of a medium inside a vessel in the cell culture using a culture vessel.

### SOLUTIONS TO THE PROBLEMS

In order to achieve the above object, a cell culture method of the present invention uses a culture vessel and a medium supplying vessel. Cells and a medium are filled and cell culture is performed in the culture vessel, a new medium is filled in the medium supplying vessel, and the medium supplying vessel supplies the medium to the culture vessel. The culture vessel includes at least a first port used for medium supply and a second port used for medium discharge, and the medium supplying vessel serves as a mixing vessel for adjusting a concentration of the new medium to be supplied to the culture vessel. The cell culture method includes: transferring a portion of a used medium in the culture vessel from the culture vessel to the mixing vessel; creating a concentration-adjusted medium by mixing the new medium and the portion of the used medium in the mixing vessel; and filling the culture vessel with the concentration-adjusted medium by discharging a remaining used medium from the culture vessel while the concentration-adjusted medium is transferred from the mixing vessel to the culture vessel.

Furthermore, the cell culture method is preferred in which an amount of medium inside the culture vessel is unchanged when the remaining used medium is discharged from the culture vessel while the concentration-adjusted medium is transferred from the mixing vessel to the culture vessel, the discharging from the culture vessel is stopped after the remaining used medium inside the culture vessel is replaced with the concentration-adjusted medium, and the concentration-adjusted medium in an amount corresponding to the portion of the used medium is transferred from the mixing vessel to the culture vessel.

Furthermore, the cell culture method is preferred in which a liquid thickness of the medium in the culture vessel is decreased by discharging a portion of the remaining used medium in the culture vessel from the culture vessel before the remaining used medium is discharged from the culture vessel while the concentration-adjusted medium is transferred from the mixing vessel to the culture vessel, and the culture vessel is filled with the concentration-adjusted medium by discharging all the rest of the remaining used medium in the culture vessel from the culture vessel while a state where the liquid thickness of the medium is decreased in the culture vessel is maintained and the concentration-adjusted medium is transferred from the mixing vessel to the culture vessel.

Furthermore, the cell culture method is preferred in which the discharging from the culture vessel is stopped after all the rest of the remaining used medium inside the culture vessel is replaced with the concentration-adjusted medium, and the concentration-adjusted medium in an amount corresponding to the portion of the used medium and the portion of the remaining used medium is transferred from the mixing vessel to the culture vessel.

Furthermore, the cell culture method is preferred in which a medium exchange rate of the culture vessel is controlled by adjusting a mixture ratio of an amount of new medium in the mixing vessel to an amount of used medium transferred from the culture vessel to the mixing vessel.

Furthermore, the cell culture method is preferred in which before the mixture ratio of the amount of new medium in the mixing vessel to the amount of used medium transferred from the culture vessel to the mixing vessel is adjusted, a liquid transfer amount required for medium exchange inside the culture vessel is measured, a desired concentration of the new medium to be supplied to the culture vessel is determined, and the amount of new medium in the mixing vessel and the amount of used medium to be transferred from the culture vessel to the mixing vessel are determined based on the required liquid transfer amount and the desired concentration.

Furthermore, the cell culture method is preferred in which the medium exchange rate of the culture vessel is controlled from 50% or more to less than 100%.

Furthermore, a cell culture kit has a configuration that includes a culture vessel, a mixing vessel, and a waste liquid vessel. The culture vessel at least includes a first port used for medium supply and a second port used for medium discharge. The culture vessel is filled with cells and a medium and used for cell culture. The mixing vessel is communicable with the first port via a tubular member. The mixing vessel is filled with a new medium and supplies the medium to the culture vessel. The waste liquid vessel is communicable with the second port via a tubular member and into which a used medium flows. The culture vessel is used to transfer a portion of the used medium in the culture vessel from the culture vessel to the mixing vessel, discharge a remaining used medium from the culture vessel to the waste liquid vessel while a concentration-adjusted medium created by mixing the new medium and the portion of the used medium in the mixing vessel is supplied from the mixing vessel, and fill the culture vessel with the concentration-adjusted medium.

Furthermore, a cell culture system has a configuration that includes a culture vessel, a mixing vessel, a waste liquid vessel, and a control device. The culture vessel at least includes a first port used for medium supply and a second port used for medium discharge. The culture vessel is filled with cells and a medium and is used for cell culture. The mixing vessel is communicated with the first port via a first tubular member. The mixing vessel is filled with a new medium and supplies the medium to the culture vessel. The waste liquid vessel is communicated with the second port via a second tubular member and into which a used medium flows. The control device controls operations of first liquid transfer means disposed in the first tubular member and second liquid transfer means disposed in the second tubular member, and controls transferring of the medium between the culture vessel, the mixing vessel, and the waste liquid vessel. The control device causes a portion of the used medium in the culture vessel to be transferred from the culture vessel to the mixing vessel and causes a remaining used medium to be transferred from the culture vessel to the waste liquid vessel while causing a concentration-adjusted medium created by mixing the new medium and the portion of the used medium in the mixing vessel to be transferred from the mixing vessel to the culture vessel.

Furthermore, the cell culture system has a preferred configuration that further includes a detection unit that measures a change in a liquid thickness of the medium. The control device controls the transferring of the medium between the culture vessel, the mixing vessel, and the waste liquid vessel based on detected information input from the detection unit.

### EFFECTS OF THE INVENTION

According to the present invention, in the cell culture using a culture vessel, it becomes possible to provide a cell culture method, a cell culture kit, and a cell culture system that can suitably control the exchange rate of a medium inside a vessel.

### BRIEF DESCRIPTION OF THE DRAWINGS

Fig. 1 is a schematic diagram illustrating a configuration of a culture vessel and a mixing vessel usable for a cell culture method according to an embodiment of the present invention.
Fig. 2 is a schematic diagram illustrating a configuration of a cell culture kit usable for the cell culture method according to the embodiment of the present invention.
Fig. 3 is a schematic diagram illustrating a configuration of a cell culture system usable for the cell culture method according to the embodiment of the present invention.
Fig. 4 is a diagram illustrating a graph of a calibration curve generated in Experiment 1 for determining an amount of a medium to be used required for a medium exchange used in the cell culture method according to the embodiment of the present invention.
Fig. 5 is a diagram illustrating a procedure of Experiment 1 for determining an amount of a medium to be used required for a medium exchange used in the cell culture method according to the embodiment of the present invention.
Fig. 6 is a diagram illustrating graphs that represent results of Experiment 1 for determining an amount of a medium to be used required for a medium exchange used in the cell culture method according to the embodiment of the present invention.
Fig. 7 is a diagram illustrating a procedure of Working Example 1 for confirming effects of the cell culture method, the cell culture kit, and the cell culture system according to the embodiment of the present invention.
Fig. 8 is a diagram illustrating a procedure of Working Example 2 for confirming the effects of the cell culture method, the cell culture kit, and the cell culture system according to the embodiment of the present invention.
Fig. 9 is a diagram illustrating a procedure of Comparative Example 2 for confirming the effects of the cell culture method, the cell culture kit, and the cell culture system according to the embodiment of the present invention.

### DESCRIPTION OF PREFERRED EMBODIMENTS

The following describes embodiments of a cell culture method, a cell culture kit, and a cell culture system of the present invention in detail. However, the present invention is not limited to the following embodiments and the specific contents of working examples described later.

The cell culture method of this embodiment is a cell culture method using a culture vessel in which cells and a medium are filled and cell culture is performed and a medium supplying vessel that is filled with a new medium (medium added to the culture vessel) and supplies the medium to the culture vessel.

The culture vessel includes a first port used for medium supply and a second port used for medium discharge. The culture vessel may include three or more ports.

In the cell culture method of this embodiment, the medium supplying vessel is used as a mixing vessel for adjusting a concentration of the new medium in the culture vessel.

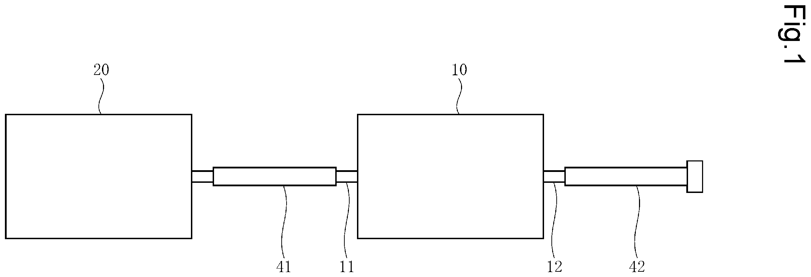

The culture vessel and the mixing vessel used in the cell culture method in this embodiment are illustrated in Fig. 1.

As illustrated in Fig. 1, by a tube 41 being coupled to a first port 11 of a culture vessel 10 and the tube 41 being coupled to a port of the mixing vessel, the culture vessel 10 is communicated with a mixing vessel 20. A tube 42 is coupled to a second port 12 of the culture vessel 10, and a used medium can be discharged to the outside of the culture vessel through this tube 42.

In Fig. 1, while a case where closed-type culture bags made of a soft packaging material are used as the culture vessel 10 and the mixing vessel 20 is illustrated, these vessels are not limited to bag-shaped vessels, and the cell culture method of this embodiment can be applied even to rigid open-type vessels.

In the cell culture method of the embodiment, first, a portion of the used medium in the culture vessel 10 is transferred to the mixing vessel 20 from the culture vessel 10 via the first port 11.

Next, in the mixing vessel 20, a concentration-adjusted medium is generated by mixing the new medium and the portion of the used medium, and a remaining used medium is discharged from the culture vessel 10 while the concentration-adjusted medium is transferred from the mixing vessel 20 to the culture vessel 10, and the culture vessel 10 is filled with the concentration-adjusted medium.

At this time, transferring of the concentration-adjusted medium from the mixing vessel 20 to the culture vessel 10 and discharging of the remaining used medium from the culture vessel 10 do not necessarily have to be simultaneously performed, and may be performed intermittently.

The amount of mediums inside the culture vessel 10 is preferred not to be changed when the remaining used medium is discharged from the culture vessel 10 while the concentration-adjusted medium is transferred from the mixing vessel 20 to the culture vessel 10. It is preferred to stop discharging from the culture vessel 10 and transfer an amount of new mediums corresponding to the portion of the used medium from the mixing vessel 20 to the culture vessel 10 after the remaining used medium inside the culture vessel 10 is replaced with the concentration-adjusted medium.

Specifically, for example, the culture vessel 10 is filled with 20 ml of the new medium and cells in advance, and the mixing vessel 20 is filled with 40 ml of the new medium, and the cells are cultured in the culture vessel 10.

After performing the culture for a predetermined period, 4.4 ml of the used medium as the portion of the used medium is transferred from the culture vessel 10 to the mixing vessel 20. At this time, the amount of medium inside the culture vessel 10 is 15.6 ml (= 20 ml - 4.4 ml).

Next, in the mixing vessel 20, 40 ml of the new medium and 4.4 ml of the portion of the used medium are mixed and the concentration-adjusted medium with a new medium concentration of 90% (volume%) (= 40/(40 + 4.4) × 100) is created.

Then, without changing the amount of mediums inside the culture vessel 10 (keeping at 15.6 ml), the remaining used medium is discharged from the culture vessel 10 while the concentration-adjusted medium is transferred from the mixing vessel 20 to the culture vessel 10, and the culture vessel 10 is filled with the concentration-adjusted medium.

After the remaining used medium inside the culture vessel 10 is replaced with the concentration-adjusted medium, discharging from the culture vessel 10 is stopped, and the concentration-adjusted medium in an amount (4.4 ml) corresponding to the portion of the used medium is transferred from the mixing vessel 20 to the culture vessel 10.

Thus, the culture vessel 10 can be filled with 20 ml of the concentration-adjusted medium having a new medium concentration of 90%. Namely, an exchange rate of the medium inside the culture vessel 10 can be controlled to 90%.

When the cell culture method of the embodiment is made into such a method, it is possible to control the exchange rate of the medium inside the vessel to any value without forming a circular flow passage or stirring the inside of the vessel in order to homogenize the medium.

In the cell culture method of the embodiment, it is preferred to discharge a portion of the remaining used medium in the culture vessel 10 from the culture vessel 10 to decrease a liquid thickness of the medium in the culture vessel 10, before discharging the remaining used medium from the culture vessel 10 while the concentration-adjusted medium is transferred from the mixing vessel 20 to the culture vessel 10.

The liquid thickness of the medium in the culture vessel 10 is the height from the bottom surface inside the vessel to the liquid surface of the medium, and when the culture vessel 10 is a bag, it corresponds to the height from the bottom surface inside the vessel to the top surface of the vessel.

At this time, the liquid thickness of the medium in the culture vessel 10 is preferably set to such an extent that cells do not flow out from the culture vessel, and when the liquid thickness is up to about 1 mm, it is possible to perform the medium exchange such that the cells do not flow out from the culture vessel.

Furthermore, it is preferred to discharge all the rest of the remaining used medium in the culture vessel 10 from the culture vessel 10 while the concentration-adjusted medium is transferred from the mixing vessel 20 to the culture vessel 10 and the culture vessel 10 is filled with the concentration-adjusted medium, while a state where the liquid thickness of the medium is decreased is maintained in the culture vessel 10.

In the cell culture method of the embodiment, it is preferred that after all the rest of the remaining used medium inside the culture vessel 10 is replaced with the concentration-adjusted medium, the discharge from the culture vessel 10 is stopped, and the concentration-adjusted medium in an amount corresponding to the portion of the used medium and the portion of the remaining used medium from the mixing vessel 20 is transferred to the culture vessel 10.

Specifically, for example, assume that a bag with a bottom area of 50 cm² and a maximum capacity of 50 ml is used as the culture vessel 10.

This culture vessel 10 is filled in advance with 20 ml of the new medium and cells, and the mixing vessel 20 is filled with 22.5 ml of the new medium, and the cells are cultured in the culture vessel 10.

As described later, since an amount of new mediums to be used can be reduced when transferring is performed with the liquid thickness of the medium in the culture vessel 10 decreased, compared with a case where the liquid thickness of the medium is not decreased, the amount of new mediums filled in the mixing vessel 20 in advance can be reduced. Thus, it is possible to further reduce the amount of new mediums required for the medium exchange.

Next, after performing the culture for a predetermined period, as the portion of the used medium, 2.5 ml of the used medium is transferred from the culture vessel 10 to the mixing vessel 20. At this time, the amount of medium inside the culture vessel 10 is 17.5 ml (= 20 ml - 2.5 ml).

Next, in the mixing vessel 20, the concentration-adjusted medium having a new medium concentration of 90% (= 22.5/(22.5 + 2.5) × 100) is created by mixing 22.5 ml of the new medium and 2.5 ml of the portion of the used medium.

Next, before discharging the remaining used medium from the culture vessel 10 while the concentration-adjusted medium is transferred from the mixing vessel 20 to the culture vessel 10, the portion of the remaining used medium in the culture vessel 10 is discharged from the culture vessel 10, and the liquid thickness of the medium in the culture vessel 10 is decreased to 1 mm. When the bottom area is 50 cm², when 12.5 ml of the used medium in the culture vessel 10 is discharged from the culture vessel 10 and an amount of used mediums in the culture vessel 10 is 5 ml, the liquid thickness of the medium will be 1 mm.

Furthermore, while the liquid thickness of the medium in the culture vessel 10 is maintained at 1 mm, all the rest of the remaining used medium in the culture vessel 10 is discharged from the culture vessel 10 while the concentration-adjusted medium is transferred from the mixing vessel 20 to the culture vessel 10, and the culture vessel 10 is filled with the concentration-adjusted medium.

After all the rest of the remaining used medium inside the culture vessel 10 is replaced with the concentration-adjusted medium, the discharging from the culture vessel 10 is stopped, and 15 ml ( = 2.5 ml + 12.5 ml) of the concentration-adjusted medium in an amount corresponding to the portion of the used medium and the portion of the remaining used medium is transferred from the mixing vessel 20 to the culture vessel 10.

Thus, the culture vessel 10 can be filled with 20 ml of the concentration-adjusted medium having a new medium concentration of 90%. Namely, the exchange rate of the medium inside the culture vessel 10 can be controlled to 90%. It is possible to further reduce the amount of new mediums required for the medium exchange.

In the cell culture method of the embodiment, it is possible to control the medium exchange rate in the culture vessel 10 by adjusting a mixture ratio of the amount of new mediums in the mixing vessel 20 to the amount of used mediums transferred from the culture vessel 10 to the mixing vessel 20.

Namely, in the above-described example, by setting the amount of new mediums in the mixing vessel 20 to 22.5 m l and setting the amount of used mediums transferred from the culture vessel 10 to the mixing vessel 20 to 2.5 ml, the concentration of the new medium in the concentration-adjusted medium is set to 90%, and the exchange rate of the medium inside the culture vessel 10 is controlled to 90%.

According to such a cell culture method of the embodiment, it is possible to control the exchange rate of the medium inside the culture vessel 10 to any value other than 90%.

Furthermore, in the cell culture method of the embodiment, from a viewpoint of cell culture efficiency, the medium exchange rate of the culture vessel is preferably controlled to 50% or more and less than 100%, and more preferably controlled to 50% or more and less than 99%.

In the cell culture method of the embodiment, before adjusting the mixture ratio of the amount of new mediums in the mixing vessel 20 to the amount of used mediums transferred from the culture vessel 10 to the mixing vessel 20, it is preferred to measure a liquid transfer amount required for the medium exchange inside the culture vessel 10, determine a desired concentration of the new medium to be supplied to the culture vessel 10, and determine the amount of new mediums in the mixing vessel 20 and the amount of used mediums transferred from the culture vessel 10 to the mixing vessel 20 based on the required liquid transfer amount and the desired concentration.

Specifically, as described in detail in Experiment 1, which will be described later, for example, by filling transparent water inside the culture vessel 10, transferring red water from the mixing vessel 20 to the culture vessel 10 and performing an operation of discharging the transparent water from the culture vessel 10 while the liquid amount of the filled transparent water is maintained to be constant, and measuring the liquid transfer amount used until the transparent water inside the culture vessel is replaced with the red water, it is possible to measure the liquid transfer amount required for the medium exchange inside the culture vessel 10.

As described in detail in Working Example 1 and Working Example 2, which will be described later, by considering the desired concentration of the new medium in the culture vessel 10, a liquid amount inside the mixing vessel 20, the liquid amount made to flow into the mixing vessel from the culture vessel, the required liquid transfer amount for the medium exchange inside the culture vessel 10, the liquid amount required for filling up the liquid amount inside the culture vessel 10 to the original amount, it is possible to determine the amount of new mediums in the mixing vessel 20 and the amount of used mediums transferred from the culture vessel 10 to the mixing vessel 20.

In the cell culture method of the embodiment, when a closed-type culture bag is used as the culture vessel 10, a resin film or the like can be suitably used as its material, and, for example, polyolefin-based resin such as polyethylene and polypropylene can be used. For example, examples of the material include polyethylene, a copolymer of ethylene and α-olefin, a copolymer of ethylene and vinyl acetate, and ionomers, or the like, in which ethylene, an acrylic acid or methacrylic acid copolymer and metal ions are used. Polyolefin, a styrene-based elastomer, a polyester-based thermoplastic elastomer, or the like can also be used. Furthermore, soft vinyl chloride resin, polybutadiene resin, an ethylene-vinyl acetate copolymer, a chlorinated polyethylene resin, a polyurethane-based thermoplastic elastomer, a polyester-based thermoplastic elastomer, a silicone-based thermoplastic elastomer, a styrene-based elastomer, for example, SBS (styrene-butadiene-styrene), SIS (styrene-isoprenoid-styrene), SEBS (styrene-ethylene-butylene-styrene), SEPS (styrene-ethylene-propylene-styrene), polyolefin resin, fluorine-based resin, or the like may be used.

In the cell culture method of the embodiment, when a rigid open-type vessel is used as the culture vessel 10, while its material is not particularly limited, for example, plastics such as polystyrene, PET, PETG, TPX, metals such as stainless steel, glass, or a combination of these, or the like can be used.

Here, when the closed-type culture bag is used as the culture vessel 10, since the top surface of the culture bag follows changes in an amount of filled liquid (liquid thickness), it is easier to control the liquid thickness compared with a case of using a rigid open-type vessel. Since the top surface of the culture bag suppresses the filled medium from undulating, stability of the liquid transfer is improved. Furthermore, since a liquid thickness detector can be attached to the top surface of the culture bag, the liquid thickness of the bag can be easily measured, and the liquid thickness can be accurately controlled.

In the cell culture method of the embodiment, the cells that are cultured using the culture vessel 10 are not particularly limited, and may be floating cells, adherent cells, spheres (cell aggregations), or organoids created by aggregating several kinds of cells.

Specifically, examples of cells can include nerve cells, pancreatic islet cells, renal cells, hepatic cells, muscle cells, cardiac muscle cells, corneal endothelial cells, vascular endothelial cells, mesenchymal stem cells, lymph corpuscles. Examples of cells can also include induced pluripotent stem cells (iPS cells), embryonic stem cells (ES cells), and cells that are differentiated and induced to the above-described various kinds of cells from those stem cells. Furthermore, it is also possible to culture intermediates such as precursor cells generated during a process of differentiation induction.

The cell culture method of the embodiment can be applied to a differentiation-induced culture in any culture of floating cells, adherent cells, spheres, or organoids.

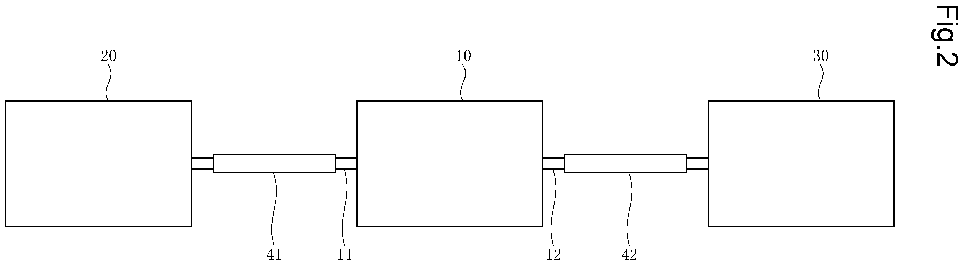

As illustrated in Fig. 2, the cell culture kit of the embodiment includes the culture vessel 10 that at least includes the first port 11 used for medium supply and the second port 12 used for medium discharge, and is filled with cells and the medium and used for the cell culture, the mixing vessel 20 that can communicate with the first port 11 via the tube 41 and is filled with the new medium to supply the medium to the culture vessel 10, and a waste liquid vessel 30 that can communicate with the second port 12 via the tube 42 and into which the used medium flows.

Then, the culture vessel 10 transfers the portion of the used medium in the culture vessel 10 to the mixing vessel 20 from the culture vessel 10, discharges the remaining used medium to the waste liquid vessel 30 from the culture vessel 10 while the concentration-adjusted medium, which is created by mixing the new medium and the portion of the used medium in the mixing vessel 20, is supplied from the mixing vessel 20, and is used to fill the culture vessel 10 with the concentration-adjusted medium.

By using such cell culture kit of the embodiment, the medium in the culture vessel 10 can be exchanged uniformly, and the exchange rate of the medium can be controlled to any value.

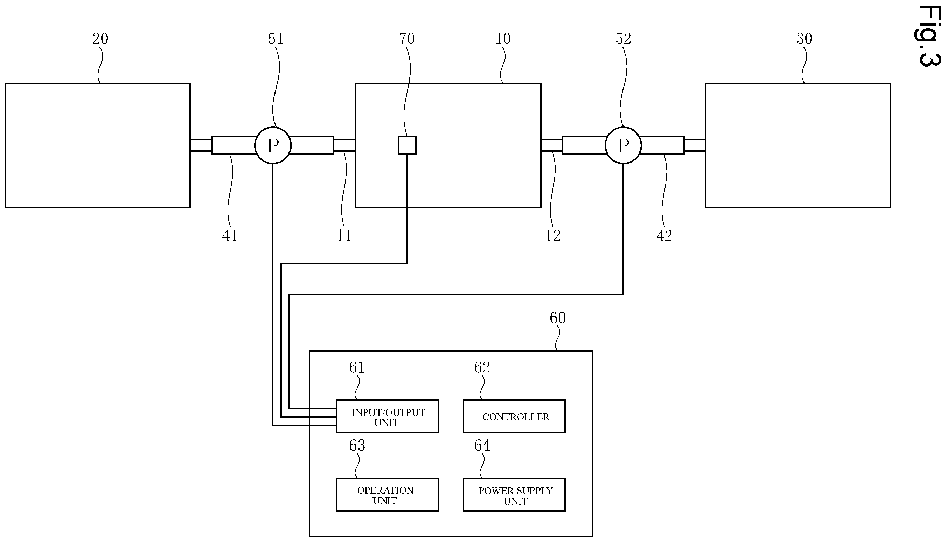

As illustrated in FIG. 3, the cell culture system of the embodiment includes the culture vessel 10 that at least includes the first port 11 used for medium supply and the second port 12 used for medium discharge, and is filled with cells and the medium and used for the cell culture, the mixing vessel 20 that is communicated with the first port 11 via the tube 41 and is filled with the new medium to supply the medium to the culture vessel 10, the waste liquid vessel 30 that is communicated with the second port 12 via the tube 42 and into which the used medium flows, and a control device 60 that controls operations of a first pump 51 disposed in the tube 41 and a second pump 52 disposed in the tube 42 to control the transferring of the medium between the culture vessel 10, the mixing vessel 20, and the waste liquid vessel 30.

Then, the control device 60 causes the portion of the used medium in the culture vessel 10 to be transferred from the culture vessel 10 to the mixing vessel 20 and causes the remaining used medium to be transferred from the culture vessel 10 to the waste liquid vessel 30 while causing the concentration-adjusted medium created by mixing the new medium and the portion of the used medium in the mixing vessel 20 to be transferred from the mixing vessel 20 to the culture vessel 10.

It is also preferred that the cell culture system of the embodiment is configured such that the system further includes a detection unit 70 that measures changes in the liquid thickness of the medium, and the control device 60 controls the transferring of the medium between the culture vessel 10, the mixing vessel 20, and the waste liquid vessel 30 based on detected information input from the detection unit 70.

Specifically, the control device 60 (a control unit) can include an input/output unit 61, a controller 62, an operation unit 63, and a power supply unit 64.

The input/output unit 61 is connected to the first pump 51 disposed between the culture vessel 10 and the mixing vessel 20 and the second pump 52 disposed between the culture vessel 10 and the waste liquid vessel 30 and controls the operation of these pumps based on input information from the controller 62. With this, the concentration-adjusted medium is supplied from the mixing vessel 20 to the culture vessel 10, and the used medium is discharged from the culture vessel 10 to the waste liquid vessel 30.

The controller 62 is constituted of a Programmable Logic Controller (PLC) or the like, can program and store desired control contents in advance, and control the operations of each unit based on this. Namely, the controller 62 transmits information for controlling the pumps to the input/output unit 61 at a predetermined timing.

The operation unit 63 includes a display unit such as a touch panel and transmits input information by a user to the controller 62 to execute setting of PLC or the like. The operation unit 63 displays the input information from the controller 62.

The power supply unit 64 (a stabilized power supply or the like) supplies electricity to each unit inside the control device 60.

While not illustrated, the control device 60 can be configured to further include a relay unit (a relay) and a wiring cutoff unit (a circuit breaker). A portion or all of the configuration of the controller 62, the operation unit 63, or the like in the control device 60 may be achieved by a microcomputer or a computer.

In the cell culture system of the embodiment, as the detection unit 70 that measures changes in the liquid thickness of the medium, for example, a length measurement sensor can be suitably used. Then, it is possible to cause the controller 62 to calculate the liquid thickness of the medium based on the detected information, and the operations of the first pump 51 and the second pump 52 can be controlled based on this liquid thickness. For example, it is possible to control to discharge the used medium from the culture vessel 10 such that the liquid thickness of the medium inside the culture vessel 10 becomes 1 mm.

According to such cell culture system of the embodiment, the medium exchange in the culture vessel 10 can be uniformly performed, the exchange rate of the medium can be controlled to any value, and it is possible to perform these automatically.

As described above, according to the cell culture method, the cell culture kit, and the cell culture system in the embodiment, in the cell culture using the culture vessel, it is possible to suitably control the exchange rate of the medium inside the vessel.

According to the embodiment, the medium exchange in the culture vessel can be performed uniformly, and the amount of used mediums can also be reduced. Furthermore, it is also possible to automatically perform such medium exchange.

### WORKING EXAMPLES

The following describes various kinds of experiments performed for confirming effects of the cell culture method, the cell culture kit, and the cell culture system according to the embodiment of the present invention.

### [Experiment 1]

First, in Working Examples 1, 2 and Comparative Example 2, which will be described later, experiments to determine the amount of mediums to be used required for the medium exchange inside the culture vessel were performed.

As a culture vessel, a closed-type culture bag which was coated with a polymer with low cell adhesion and in which 18,000 hemispherical wells with a diameter of 0.5 mm were formed on the bottom surface (the culture surface) inside the vessel was used. The bag bottom area was 50 cm², the maximum liquid capacity was 50 mL, and the bag material was LLDPE (the linear low-density polyethylene). The culture vessel included two ports.

As a mixing vessel, a bag with a flat bottom surface inside the vessel without any molded well was used. The bag bottom area was 50 cm², the maximum liquid capacity was 50 mL, and the bag material was LLDPE. The mixing vessel included one port.

By filling the culture vessel with transparent water and performing an operation of transferring red water from the mixing vessel to the culture vessel and discharging the transparent water from the culture vessel while the liquid amount of the filled transparent water was maintained to be constant, the liquid transfer amount used until the transparent water inside the culture bag was replaced with the red water was measured.

Specifically, the liquid transfer amount was measured when 15.6 mL (liquid thickness 3.1 mm) of transparent water was filled into the culture vessel and when 5 mL (liquid thickness 1 mm) of the transparent water was filled into the culture vessel.

As a preliminary preparation for the experiment, a calibration curve was created based on a relationship between a red water concentration and absorbance.

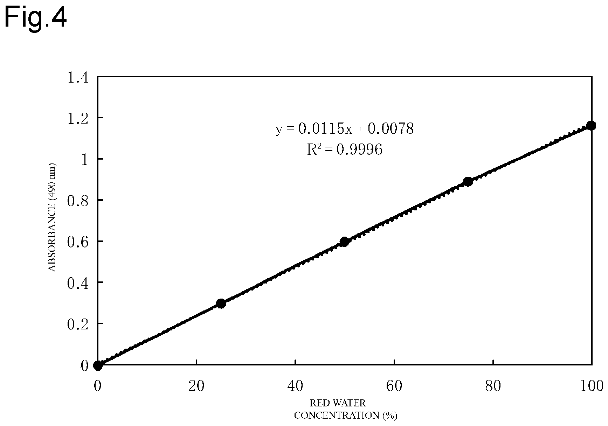

Specifically, a red water undiluted solution (100%), red water 75%, red water 50%, red water 25%, and transparent water (0%, control) were prepared, and 200 µL of each was placed in 96-well plates. Then absorbance measurement (wavelength 490 nm) was performed using an absorbance plate reader (SH-1000 manufactured by Corona Electric Co., Ltd.), and the calibration curve was created from the relationship between the red water concentration and the absorbance. The results are illustrated in Fig. 4. In the following experiment, the red water concentration was calculated from the absorbance of the red water using this calibration curve.

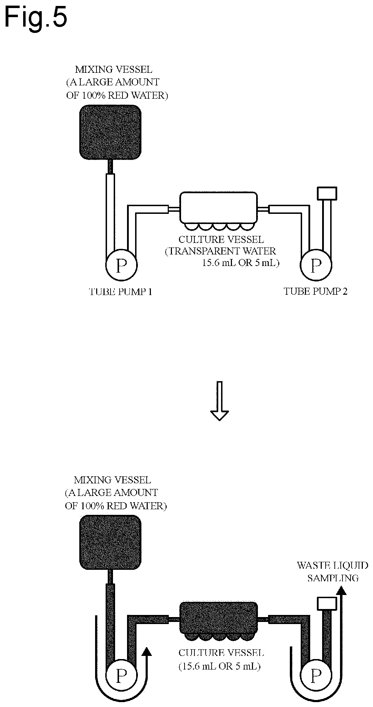

As for an experimental method, as illustrated in Fig. 5, the culture vessel was filled with 15.6 mL or 5 mL of the transparent water, and the mixing vessel was filled with a sufficient amount (50 mL) of the red water undiluted solution (100%). Then, a tube was coupled to one port of the culture vessel to cause the mixing vessel to communicate with the culture vessel. A tube was coupled to the other port of the culture vessel, and a setting was made to perform sampling of the waste liquid. A tube pump was disposed in each tube.

Then, by operating both tube pumps and simultaneously performing the liquid transfer in a waste liquid direction at an identical rate of 1 mL/min, the liquid transfer was performed while the liquid amount and the liquid thickness inside the culture vessel were maintained to be constant. The sampling of the waste liquid was performed every 5 minutes.

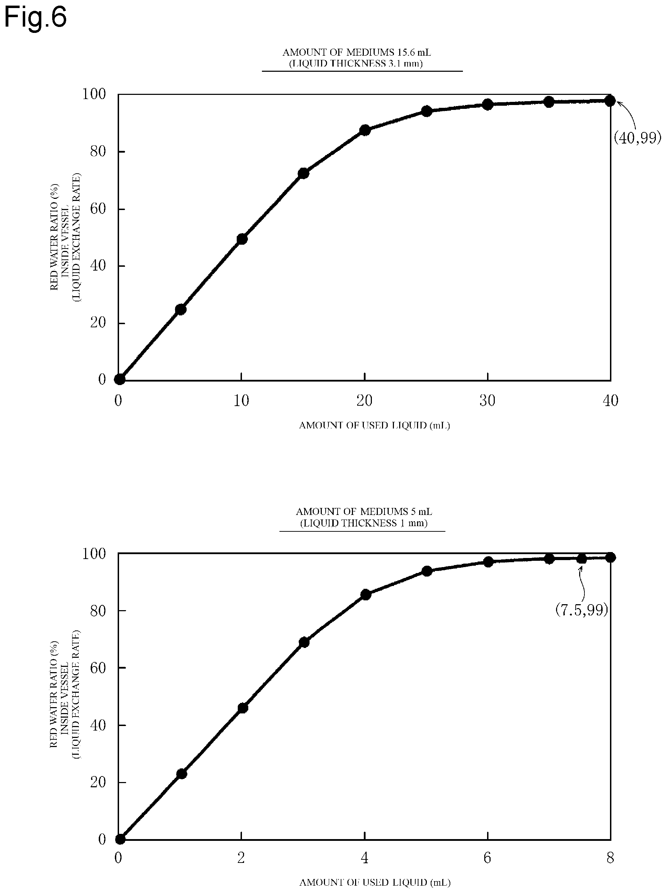

Next, the absorbance measurement of the sampled waste liquid was performed to calculate the red water concentration and a transparent water concentration of the waste liquid. Based on the transparent water concentration and the amount of waste liquid, the transparent water discharged every 5 minutes of liquid transfer was calculated to calculate a liquid exchange rate relative to the amount of used red water (a ratio of red water inside the vessel). The results are illustrated in Fig. 6.

In Fig. 6, the amount of used liquid was confirmed at a timing when 99% of the inside of the vessel was replaced with the red water. As a result, when the liquid amount inside the culture vessel was set to 15.6 mL (the liquid thickness 3.1 mm) and the liquid transfer was performed while the liquid amount and the liquid thickness inside the culture vessel were maintained to be constant, it was found that the liquid transfer amount required for the liquid inside the culture vessel to be replaced with the liquid filled inside the mixing vessel was 40 mL.

Similarly, when the liquid amount inside the culture vessel was set to 5 mL (the liquid thickness 1 mm) and the liquid transfer was performed while the liquid amount and the liquid thickness inside the culture vessel were maintained to be constant, it was found that the liquid transfer amount required for the liquid inside the culture vessel to be replaced with the liquid filled inside the mixing vessel was 7.5 mL.

### [Working Example 1]

Regarding a method of transferring a portion of the used medium in the culture vessel from the culture vessel to the mixing vessel, creating the concentration-adjusted medium by mixing the new medium and the portion of the used medium in the mixing vessel, discharging the remaining used medium from the culture vessel while the concentration-adjusted medium is transferred from the mixing vessel to the culture vessel, and filling the culture vessel with the concentration-adjusted medium, an experiment for confirming its effect was performed.

As the culture vessel and the mixing vessel, the same ones as those used in Experiment 1 were used. As the waste liquid vessel, the same one as the mixing vessel was used.

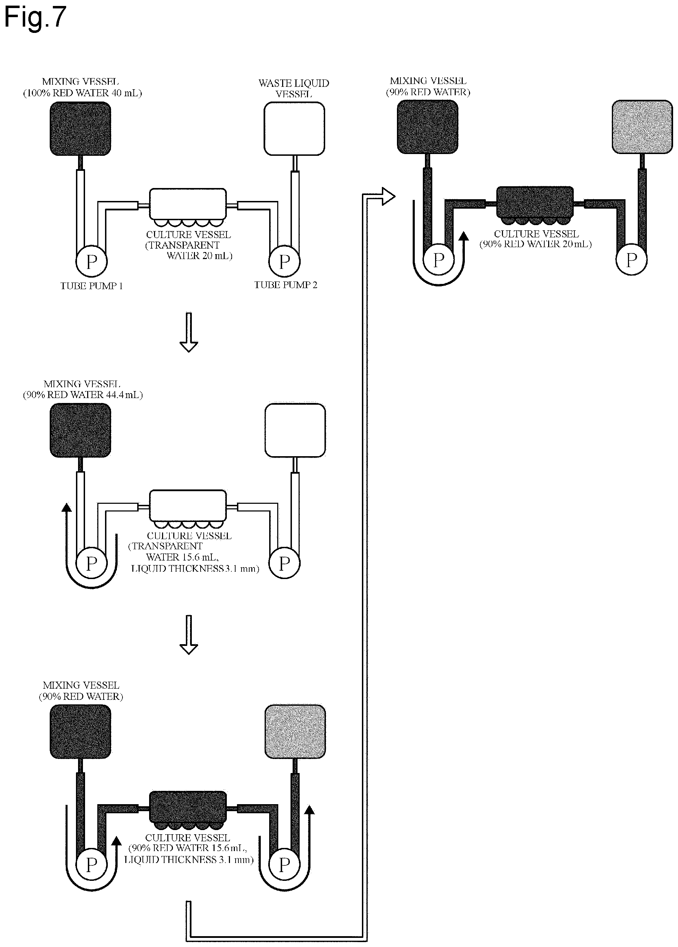

As an experiment method, as illustrated in Fig. 7, the culture vessel was filled with 20 mL of the transparent water, and the mixing vessel was filled with 40 mL of the red water undiluted solution (100%). Then, a tube was coupled to one port of the culture vessel to cause the mixing vessel to communicate with the culture vessel, and a tube was coupled to the other port of the culture vessel to cause the waste liquid vessel to communicate with the culture vessel.

A tube pump 1 was disposed on the tube coupling the mixing vessel to the culture vessel, and a tube pump 2 was disposed on the tube coupling the waste liquid vessel to the culture vessel. The transparent water inside the culture vessel was set to be exchanged for the red water having a concentration of 90%.

Then, by operating the tube pump 1, 4.4 mL of the transparent water inside the culture vessel was made to flow into the mixing vessel. With this, the red water having a concentration of 90% was prepared inside the mixing vessel. At this time, a liquid transfer rate was set to 1 mL/min, and a liquid transfer time was 4.4 minutes. In the following experiments, the liquid transfer rate was also all set to 1 mL/min.

Here, the reason for setting a filling amount to the mixing vessel at 40 mL is that since the liquid amount inside the mixing vessel becomes +4.4 mL when 4.4 mL is made to flow into the mixing vessel from the culture vessel and becomes -40 mL from the results in Experiment 1 when the liquid inside the culture vessel is replaced with the red water and becomes -4.4 mL when the liquid amount inside the culture vessel is filled up to 20 mL, the liquid transfer can be performed as long as the filling amount is 40 mL or more.

Next, by operating both tube pumps and simultaneously performing the liquid transfer in the waste liquid direction, the liquid transfer was performed while the liquid amount and the liquid thickness inside the culture vessel were maintained to be constant, and the transparent water inside the culture vessel was uniformly replaced with the red water having a concentration of 90%. At this time, the liquid transfer time was 40 minutes.

Furthermore, the tube pump 2 was stopped to stop discharging from the culture vessel, and the tube pump 1 was operated to cause 4.4 mL of the red water having a concentration of 90% inside the mixing vessel to flow into the culture vessel, and thus, the inside of the culture vessel was filled with 20 mL of the red water having a concentration of 90%. At this time, the liquid transfer time was 4.4 minutes.

With this, it was possible to exchange the inside of the culture vessel for the red water having a concentration of 90%, and it was possible to control the medium exchange rate of the culture vessel.

Namely, according to the cell culture method of the embodiment indicated in Working Example 1, the medium exchange could be performed uniformly, and the medium exchange rate of the culture vessel could be appropriately controlled. The medium exchange time was 48.8 minutes (= 4.4 + 40 + 4.4), and this could be performed in a shorter time compared with the conventional circulation method (Comparative Example 3).

### [Working Example 2]

Regarding a method of discharging a portion of a remaining used medium in the culture vessel from the culture vessel and decreasing the liquid thickness of the medium in the culture vessel before discharging the remaining used medium from the culture vessel while the concentration-adjusted medium is transferred from the mixing vessel to the culture vessel, discharging all the rest of the remaining used medium in the culture vessel while a state where the liquid thickness of the medium is decreased in the culture vessel is maintained and the concentration-adjusted medium is transferred from the mixing vessel to the culture vessel, and filling the culture vessel with the concentration-adjusted medium, an experiment for confirming its effect was performed.

As the culture vessel and the mixing vessel, the same ones as those used in Experiment 1 were used. As the waste liquid vessel, the same one as the mixing vessel was used.

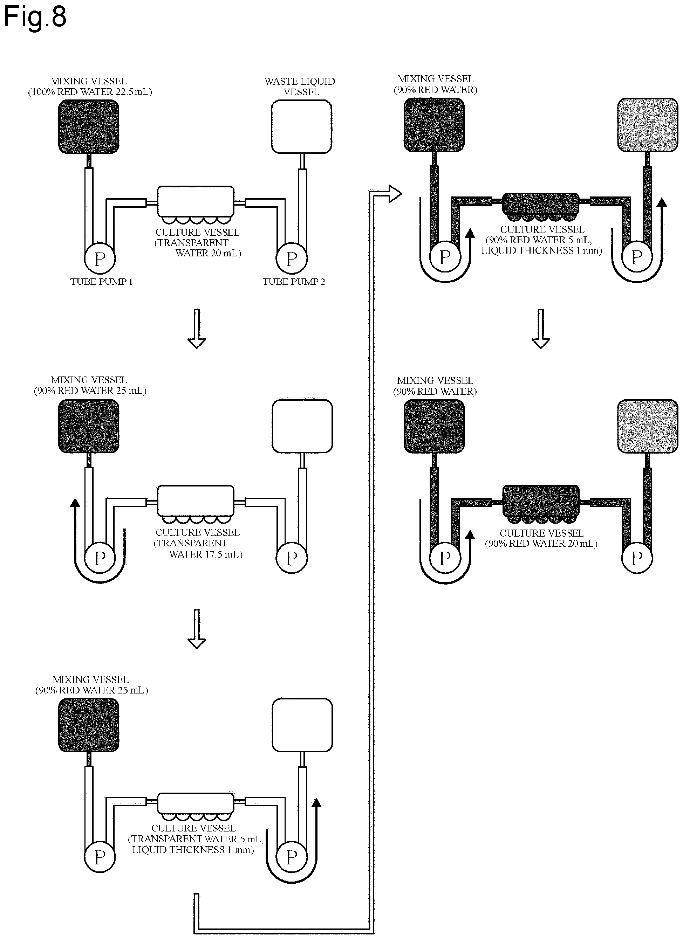

As an experiment method, as illustrated in Fig. 8, the culture vessel was filled with 20 mL of the transparent water, and the mixing vessel was filled with 22.5 mL of the red water undiluted solution (100%). Then, a tube was coupled to one port of the culture vessel to cause the mixing vessel to communicate with the culture vessel, and a tube was coupled to the other port of the culture vessel to cause the waste liquid vessel to communicate with the culture vessel.

The tube pump 1 was disposed on the tube coupling the mixing vessel to the culture vessel, and the tube pump 2 was disposed on the tube coupling the waste liquid vessel to the culture vessel. The transparent water inside the culture vessel was set to be exchanged for the red water having a concentration of 90%.

Then, by operating the tube pump 1, 2.5 mL of the transparent water inside the culture vessel was made to flow into the mixing vessel. With this, the red water having a concentration of 90% was prepared inside the mixing vessel. At this time, the liquid transfer time was 2.5 minutes.

Next, by stopping the tube pump 1 and operating the tube pump 2, 12.5 mL of the transparent water inside the culture vessel was transferred to the waste liquid vessel to decrease the liquid amount inside the culture vessel to 5 mL (liquid thickness 1 mm). At this time, the liquid transfer time was 12.5 minutes.

Here, the reason for having set the filling amount to the mixing vessel at 22.5 mL is that since the liquid amount inside the mixing vessel becomes +2.5 mL when 2.5 mL is made to flow into the mixing vessel from the culture vessel and becomes -7.5 mL from the results in Experiment 1 when the liquid inside the culture vessel is replaced with the red water and becomes -15 mL when the liquid amount inside the culture vessel is filled up to 20 mL, the liquid transfer can be performed as long as the filling amount is 20 mL or more.

Next, by operating both tube pumps and simultaneously performing the liquid transfer in the waste liquid direction, the liquid transfer was performed while the liquid amount and the liquid thickness inside the culture vessel were maintained to be constant (a state where the liquid thickness of the medium was decreased in the culture vessel), and the transparent water inside the culture vessel was uniformly replaced with the red water having a concentration of 90%. At this time, the liquid transfer time was 7.5 minutes.

Furthermore, the tube pump 2 was stopped to stop the discharging from the culture vessel, and the tube pump 1 was operated to cause 15 mL of the red water having a concentration of 90% inside the mixing vessel to flow into the culture vessel, and thus, the inside of the culture vessel was filled with 20 mL of the red water having a concentration of 90%. At this time, the liquid transfer time was 15 minutes.

With this, it was possible to exchange the inside of the culture vessel for the red water having a concentration of 90%, and it was possible to control the medium exchange rate of the culture vessel.

Namely, according to the cell culture method of the embodiment indicated in Working Example 2, the amount of mediums used was very small at 22.5 mL, and the medium exchange could be performed uniformly, and the medium exchange rate of the culture vessel could be appropriately controlled. The medium exchange time was 37.5 minutes (= 2.5 + 7.5 + 15), and this could be performed in a shorter time compared with the conventional circulation method (Comparative Example 3).

### [Comparative Example 1]

Regarding a method of causing a medium supplying vessel to communicate with a culture vessel by a tube to supply a new medium from the medium supplying vessel to the culture vessel, experiments for confirming its effect were performed.

As a culture vessel, a closed-type culture bag which was coated with a polymer with low cell adhesion and in which 18,000 hemispherical wells with a diameter of 0.5 mm were formed on the bottom surface (the culture surface) inside the vessel was used. The bag bottom area was 50 cm², the maximum liquid capacity was 50 mL, and the bag material was LLDPE. The culture vessel included one port.

As a medium supplying vessel, a bag with a flat bottom surface inside the vessel without any molded well was used. The bag bottom area was 50 cm², the maximum liquid capacity was 50 mL, and the bag material was LLDPE. The medium supplying vessel included one port.

As for an experimental method, the culture vessel was filled with 20 mL of the transparent water, and the medium supplying vessel was filled with 18 mL of the red water undiluted solution (100%). Then, a tube was coupled to a port of the culture vessel, and a tube pump was disposed in this tube and operated to discharge 18 mL of the transparent water from the culture vessel. At this time, the liquid transfer time was 18 minutes.

Next, the medium supplying vessel was coupled to the tube, and the tube pump was operated to transfer 18 mL of the red water undiluted solution (100%) from the medium supplying vessel to the culture vessel. At this time, the liquid transfer time was 18 minutes.

As a result, while the transparent water inside the culture vessel could be exchanged for the red water having a concentration of 90%, the red water inside the culture vessel was not uniform, and it turned out that this method could not appropriately perform the medium exchange.

### [Comparative Example 2]

Unlike Working Example 1, regarding a method of discharging the used medium from the culture vessel while the new medium is transferred from the medium supplying vessel to the culture vessel without creating the concentration-adjusted medium by mixing the new medium and the portion of the used medium in the mixing vessel, an experiment for confirming its effect was performed. Similarly to Working Example 2, the liquid transfer was performed after the liquid thickness inside the culture vessel was decreased.

As the culture vessel, the same one as that used in Experiment 1 was used. As the medium supplying vessel and the waste liquid vessel, the same ones as the mixing vessel used in Experiment 1 were used.

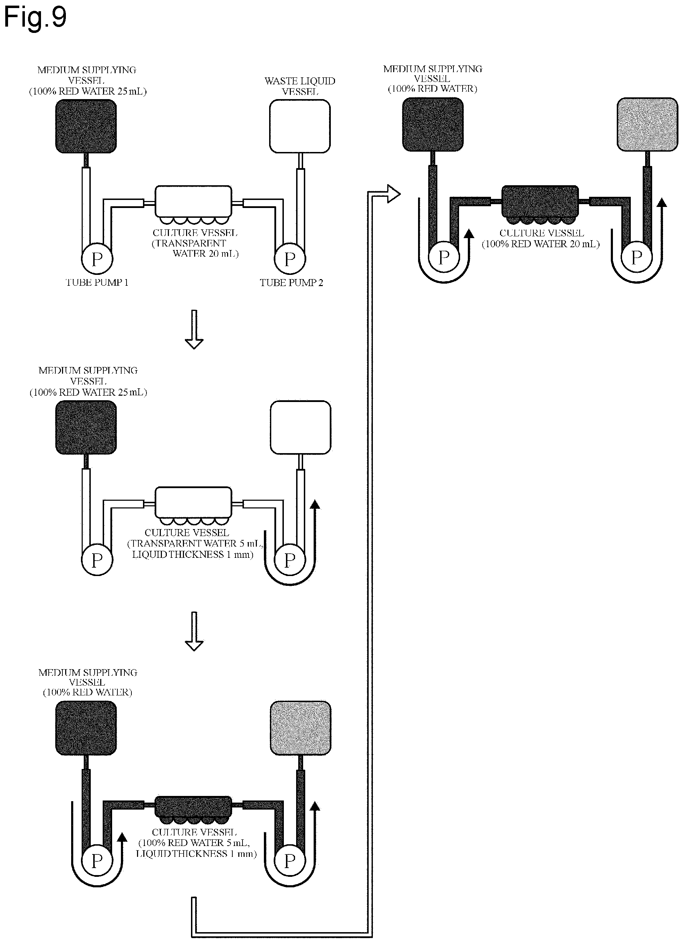

As an experiment method, as illustrated in Fig. 9, the culture vessel was filled with 20 mL of the transparent water, and the medium supplying vessel was filled with 25 mL of the red water undiluted solution (100%). Then, a tube was coupled to one port of the culture vessel to cause the medium supplying vessel to communicate with the culture vessel, and a tube was coupled to the other port of the culture vessel to cause the waste liquid vessel to communicate with the culture vessel.

The tube pump 1 was disposed on the tube coupling the medium supplying vessel to the culture vessel and the tube pump 2 was disposed on the tube coupling the waste liquid vessel to the culture vessel.

Then, by operating the tube pump 2, 15 mL of the transparent water inside the culture vessel was transferred to the waste liquid vessel to decrease the liquid amount inside the culture vessel to 5 mL (the liquid thickness 1 mm). At this time, the liquid transfer time was 15 minutes.

Here, the reason for having set the filling amount to the medium supplying vessel at 25 mL is that since the liquid amount inside the medium supplying vessel becomes -7.5 mL from the results in Experiment 1 when the liquid inside the culture vessel is replaced with the red water and becomes -15 mL when the liquid amount inside the culture vessel is filled up to 20 mL, the liquid transfer can be performed as long as the filling amount is 22.5 mL or more.

Next, by operating both tube pumps and simultaneously performing the liquid transfer in the waste liquid direction, the liquid transfer was performed while the liquid amount and the liquid thickness inside the culture vessel were maintained to be constant (a state where the liquid thickness of the medium was decreased in the culture vessel), and the transparent water inside the culture vessel was uniformly replaced with the red water having a concentration of 100%. At this time, the liquid transfer time was 7.5 minutes.

Furthermore, the tube pump 2 was stopped to stop the discharging from the culture vessel, and the tube pump 1 was operated to cause 15 mL of the red water having a concentration of 100% inside the medium supplying vessel to flow into the culture vessel, and thus, the inside of the culture vessel was filled with 20 mL of the red water having a concentration of 100%. At this time, the liquid transfer time was 15 minutes.

As a result, in the method of Comparative Example 2, while it is possible to exchange the medium inside the culture vessel at an exchange rate of 100%, it turned out that the medium exchange rate was not able to be arbitrarily controlled, and the object of the present invention was not able to be achieved.

### [Comparative Example 3]

Regarding a circulation method, which is a conventional medium exchange method, in which a medium inside a vessel is exchanged by circulating it, an experiment for confirming its effect was performed.

As a culture vessel, the same one as that used in Experiment 1 was used.

As an experiment method, first, the culture vessel was filled with 20 mL of the transparent water. Then, a tube was coupled to one port of the culture vessel, and a tube pump was disposed on this tube, and the tube pump was operated to discharge 14 mL of the transparent water from the culture vessel. At this time, the liquid transfer time was 14 minutes.

The tube pump was operated to supply 14 mL of the red water to the culture vessel via the tube. At this time, the liquid transfer time was 14 minutes.

At this time, the concentration of the red water inside the culture vessel was 70%, and the red water inside the culture vessel was not uniform.

Here, the reason for discharging 70% (14 mL) of the transparent water and setting the medium exchange rate to 70% without discharging 90% (18 mL) of the transparent water in the culture vessel and setting the medium exchange rate to 90% is that, in a case of this circulation method, the cells will be discharged together with the medium when 90% of the transparent water is discharged.

Therefore, next, one tube was coupled to two ports of culture vessel to form a circular flow passage, and a tube pump was disposed on the tube.

Then, by operating the tube pump and circulating and transferring the red water having a concentration of 70% inside the culture vessel, homogenization of the uneven liquid was performed. By circulating it for 40 minutes, the red water having a concentration of 70% inside the culture vessel was homogenized.

Next, by repeating the above-described operations from discharging 14 mL of the transparent water from the culture vessel to circulating and transferring the red water inside the culture vessel again, the inside of the culture vessel could be replaced with the red water having a concentration of 91% (= (7/10) × (3/10) + 1 × (7/10) × 100).

Namely, according to the method of Comparative Example 3, while it is possible to control the exchange rate of the medium, since it takes a long time of 136 minutes (= 14 + 14 + 40 + 14 + 14 + 40) to exchange the medium, and it is required to repeatedly perform the circulating and transferring of the liquid, there was a problem that it was complicated.

### [Reference Example 1]

An experiment was performed to confirm whether or not the cell culture efficiency is inhibited when near 100% medium exchange is performed when spheres are differentiated and induced into mesoderm.

Specifically, iPS cells (1231A3 strain, The Center for iPS Cell Research and Application, Kyoto University) were used as cells to be differentiated and induced. An ultra-low adhesion surface 6-well plate (Corning Incorporated) was used as a culture vessel.

Furthermore, as the medium, on a first day, StemFit AK02N medium (Ajinomoto Healthy Supply Co., Inc.) + 10 µM Y-27623 (FUJIFILM Wako Pure Chemical Corporation) was used, and on a second day, RPMI Medium 1640 (Thermo Fisher Scientific Inc.) + B-27 Minus Insulin (Thermo Fisher Scientific Inc.) + 6 µM CHIR99021 (FUJIFILM Wako Pure Chemical Corporation) was used.

Accumax solution (Sigma-Aldrich Co. LLC) was used as a reagent to break up cell aggregates into single cells.

As an experiment method, on a first day, iPS cells were seeded into five ultra-low adhesion surface 6-well plates at a density of 6.0 × 10⁵ cells/well, respectively. On a second day, the medium exchange was performed such that the medium exchange rates were 90%, 95%, 99%, 99.75%, and 100% for each plate, respectively. Then, on a third day, the cell aggregations were collected into a 15 mL tube and washed, then 1 mL of Accumax solution was added, heated at 37°C for 3 minutes, and then the spheres were broken up until they became single cells by pipetting. Then each of counts of single cells was measured using a hemocytometer.

As a result, as indicated in the table below, the number of cells after culture decreased at a medium exchange rate of 90% or more, and in particular, a rapid decrease was observed between 99% and 99.75%.

**[Table 1]**

| | | | | | |
|---|---|---|---|---|---|
| Medium Exchange Rate | 90% | 95% | 99% | 99.75% | Near 100% |
| Number of Cells After Culture (Cells) | 1,760 thousand | 1,655 thousand | 1,420 thousand | 790 thousand | 860 thousand |

From this, it was confirmed that when spheres are differentiated and induced into mesoderm, the culture efficiency of cells is inhibited when the medium exchange close to 100% is performed.

Namely, by cell culture method of the embodiment, it was confirmed that it is considerably important to be able to control the medium exchange rate to less than 100%, in particular less than 99%.

It is needless to say that the present invention is not limited to the above-described embodiments and working examples, and that various kinds of modifications can be made within the scope of the present invention. For example, it is needless to say that the capacity of the culture vessels and mixing vessels to be used is not limited to those used in the working examples, and it is possible to apply the present invention to medium exchange using culture vessels with various capacities and shapes.

### INDUSTRIAL APPLICABILITY

In cell culture using a culture vessel, the present invention can be suitably utilized when it is desired to control the exchange rate of the medium inside the vessel to any value.

The contents of the documents described in this specification and the specification of the Japanese application on which the Paris priority of this application is based are all incorporated herein by reference.

### DESCRIPTION OF REFERENCE SIGNS

10 culture vessel
11, 12 port
20 mixing vessel (medium supplying vessel)
30 waste liquid vessel
41, 42 tube (tubular member)
51, 52 pump (liquid transfer means)
60 control device
61 input/output unit
62 controller
63 operation unit
64 power supply unit
70 detection unit

## Claims

1. A cell culture method using a culture vessel and a medium supplying vessel, wherein cells and a medium are filled and cell culture is performed in the culture vessel, a new medium is filled in the medium supplying vessel, and the medium supplying vessel supplies the medium to the culture vessel, wherein
the culture vessel includes at least a first port used for medium supply and a second port used for medium discharge, and the medium supplying vessel serves as a mixing vessel for adjusting a concentration of the new medium to be supplied to the culture vessel, wherein
the cell culture method comprises:
transferring a portion of a used medium in the culture vessel from the culture vessel to the mixing vessel;
creating a concentration-adjusted medium by mixing the new medium and the portion of the used medium in the mixing vessel; and
filling the culture vessel with the concentration-adjusted medium by discharging a remaining used medium from the culture vessel while the concentration-adjusted medium is transferred from the mixing vessel to the culture vessel.

2. The cell culture method according to claim 1, wherein
an amount of medium inside the culture vessel is unchanged when the remaining used medium is discharged from the culture vessel while the concentration-adjusted medium is transferred from the mixing vessel to the culture vessel, the discharging from the culture vessel is stopped after the remaining used medium inside the culture vessel is replaced with the concentration-adjusted medium, and the concentration-adjusted medium in an amount corresponding to the portion of the used medium is transferred from the mixing vessel to the culture vessel.

3. The cell culture method according to claim 1, wherein
a liquid thickness of the medium in the culture vessel is decreased by discharging a portion of the remaining used medium in the culture vessel from the culture vessel before the remaining used medium is discharged from the culture vessel while the concentration-adjusted medium is transferred from the mixing vessel to the culture vessel, and
the culture vessel is filled with the concentration-adjusted medium by discharging all the rest of the remaining used medium in the culture vessel from the culture vessel while a state where the liquid thickness of the medium is decreased in the culture vessel is maintained and the concentration-adjusted medium is transferred from the mixing vessel to the culture vessel.

4. The cell culture method according to claim 3, wherein
the discharging from the culture vessel is stopped after all the rest of the remaining used medium inside the culture vessel is replaced with the concentration-adjusted medium, and the concentration-adjusted medium in an amount corresponding to the portion of the used medium and the portion of the remaining used medium is transferred from the mixing vessel to the culture vessel.

5. The cell culture method according to any one of claims 1 to 4, wherein
a medium exchange rate of the culture vessel is controlled by adjusting a mixture ratio of an amount of new medium in the mixing vessel to an amount of used medium transferred from the culture vessel to the mixing vessel.

6. The cell culture method according to claim 5, wherein
before the mixture ratio of the amount of new medium in the mixing vessel to the amount of used medium transferred from the culture vessel to the mixing vessel is adjusted,
a liquid transfer amount required for medium exchange inside the culture vessel is measured, a desired concentration of the new medium to be supplied to the culture vessel is determined, and the amount of new medium in the mixing vessel and the amount of used medium to be transferred from the culture vessel to the mixing vessel are determined based on the required liquid transfer amount and the desired concentration.

7. The cell culture method according to claim 5 or 6, wherein
the medium exchange rate of the culture vessel is controlled from 50% or more to less than 100%.

8. A cell culture kit comprising:
a culture vessel that at least includes a first port used for medium supply and a second port used for medium discharge, the culture vessel being filled with cells and a medium and being used for cell culture;
a mixing vessel that is communicable with the first port via a tubular member, the mixing vessel being filled with a new medium and supplying the medium to the culture vessel; and
a waste liquid vessel that is communicable with the second port via a tubular member and into which a used medium flows, wherein
the culture vessel is used to transfer a portion of the used medium in the culture vessel from the culture vessel to the mixing vessel, discharge a remaining used medium from the culture vessel to the waste liquid vessel while a concentration-adjusted medium created by mixing the new medium and the portion of the used medium in the mixing vessel is supplied from the mixing vessel, and fill the culture vessel with the concentration-adjusted medium.

9. A cell culture system comprising:
a culture vessel that at least includes a first port used for medium supply and a second port used for medium discharge, the culture vessel being filled with cells and a medium and being used for cell culture;
a mixing vessel that is communicated with the first port via a first tubular member, the mixing vessel being filled with a new medium and supplying the medium to the culture vessel;
a waste liquid vessel that is communicated with the second port via a second tubular member and into which a used medium flows; and
a control device that controls operations of first liquid transfer means disposed in the first tubular member and second liquid transfer means disposed in the second tubular member, and controls transferring of the medium between the culture vessel, the mixing vessel, and the waste liquid vessel, wherein
the control device causes a portion of the used medium in the culture vessel to be transferred from the culture vessel to the mixing vessel and causes a remaining used medium to be transferred from the culture vessel to the waste liquid vessel while causing a concentration-adjusted medium created by mixing the new medium and the portion of the used medium in the mixing vessel to be transferred from the mixing vessel to the culture vessel.

10. The cell culture system according to claim 9, further comprising
a detection unit that measures a change in a liquid thickness of the medium, wherein
the control device controls the transferring of the medium between the culture vessel, the mixing vessel, and the waste liquid vessel based on detected information input from the detection unit.
